# EUROPÄISCHE PATENTSCHRIFT

(11) **EP 2 478 871 B1**
(45) Veröffentlichungstag und Bekanntmachung des Hinweises auf die Patenterteilung: **13.04.2016**
(21) Anmeldenummer: 12151407.9
(22) Anmeldetag: 17.01.2012
(51) Int. Cl.: A61F 2/24, A61F 2/06

(54) **Gefäßprothese mit integrierter Aortenklappe**
Vascular prosthetic with integrated aorta valve
Prothèse vasculaire dotée d'une valvule aortique intégrée

(30) Priorität: 21.01.2011 DE 102011009555
(43) Veröffentlichungstag der Anmeldung: 25.07.2012
(73) Patentinhaber: Aesculap AG, 78532 Tuttlingen/Donau (DE)
(72) Erfinder: Goldmann, Helmut, 34119 Kassel (DE); Merckle, Christof, 68199 Mannheim (DE); Probst, Dietmar, 34212 Melsungen (DE); Brabsche, Jan-Philip, 34253 Lohfelden (DE)
(74) Vertreter: Patentanwälte Ruff, Wilhelm, Beier, Dauster & Partner mbB

(56) Entgegenhaltungen:
- WO-A1-2005/034812
- DE-A1- 10 050 099
- US-A1- 2003 187 500
- US-A1- 2007 162 103
- US-A1- 2008 288 055
- US-A1- 2010 121 423

## Beschreibung

Die Erfindung betrifft eine rohrförmige Gefäßprothese mit integrierter Aortenklappe sowie ein Verfahren zur Herstellung solcher Gefäßprothesen.

Bei dem Ersatz einer defekten Herzklappe, insbesondere der sogenannten Aortenklappe, handelt es sich in der Zwischenzeit um eine übliche Operation in der Herzchirurgie. Für solche Operationen stehen in der Regel mechanische oder biologische Herzklappen zur Verfügung.

Die üblicherweise eingesetzten Herzklappen besitzen eine ganze Reihe von Nachteilen. Dabei handelt es sich beispielsweise um die Gefahr einer Degeneration der Klappe, insbesondere durch Verkalkung, um die häufige Notwendigkeit lebenslang gerinnungshemmende Medikamente einnehmen zu müssen, um die häufige Notwendigkeit einer Reoperation bei biologischen Herzklappen sowie um die störenden Klappengeräusche bei mechanischen Herzklappen.

Wenn neben der Herzklappe, beispielsweise der Aortenklappe auch die Aorta ascendens geschädigt ist, beispielsweise durch ein Aneurysma, so muss auch diese durch eine geeignete Prothese ersetzt werden.

In diesem Zusammenhang sind deshalb bereits Implantate bekannt, welche neben der Herzklappe (z.B. Aortenklappe) zusätzlich eine Gefäßprothese aufweisen. Solche Prothesen werden auch als Conduit-Prothesen bezeichnet. Chirurgische Eingriffe, bei denen solche Prothesen eingesetzt werden, sind auch unter dem Begriff der sogenannten Bentall-Operation zu finden.

Die kombinierten Conduit-Prothesen werden dabei entweder dem Chirurgen bereits als fertiges Implantat zur Verfügung gestellt oder wahlweise vom Chirurgen selbst während der Operation aus den entsprechenden Bestandteilen zusammengefügt.
Im Ergebnis bestehen solche Conduit-Prothesen dann in der Regel aus einer mechanischen oder biologischen Herzklappe sowie einer Gefäßprothese, vorzugsweise einer gewebten Gefäßprothese.

Eine Conduit-Prothese der beschriebenen Art ist bereits bekannt aus der DE-A1-10050099, die weitgehend inhaltsgleich ist mit der US 2003/187500 A1. Diese Prothese besteht aus einem zylindrischen oder mit bulbenförmigen Ausbuchtungen versehenen Schlauch, der zwingend ein integriertes Stützgehäuse zur Stabilisierung des Annulusbereichs aufweist.

Die EP-A2-1579827 zeigt eine Conduit-Prothese, bei der an eine gewebte oder gewirkte Gefäßprothese aus Polyester Klappensegel aus Polyurethanvlies angeformt sind.

Demgegenüber sind in der WO 2005/034812 A1 und in der US 2008/0288055 A1 nur sogenannte Stents, die eine Aortenklappe aufweisen, beschrieben. Die entsprechenden Stents werden intraluminal eingeschoben, und besitzen nicht die (zusätzliche) Funktion einer Gefäßprothese. Es handelt sich also bei diesen Stents nicht um Conduit-Prothesen der zuvor beschriebenen Art.

Nachteilig bei den bekannten kombinierten Prothesen ist es jedoch, dass unter anderem häufig keine ungestörte Bewegung der Klappensegel möglich ist. Außerdem ist die Ausbildung der Klappensegel und die Verbindung der ausgebildeten Klappe mit der Gefäßprothese in der Regel sehr aufwendig.

Dementsprechend stellt sich die Erfindung die Aufgabe, eine Conduit-(Herzklappen)-Prothese mit einem vergleichsweise einfachen Aufbau zur Verfügung zu stellen. Zusätzlich soll eine solche Prothese ohne größeren Aufwand herstellbar und für den Chirurgen einfach einsetzbar sein.

Diese Aufgabe wird gelöst durch die rohrförmige Gefäßprothese mit integrierter Aortenklappe mit den Merkmalen des Anspruchs 1 sowie durch das Verfahren mit den Merkmalen des Anspruchs 7. Bevorzugte Ausführungen dieser Prothese bzw. dieses Verfahrens sind in den abhängigen Ansprüchen beschrieben. Der Wortlaut sämtlicher Ansprüche wird hiermit durch Bezugnahme zum Inhalt dieser Beschreibung gemacht.

Die erfindungsgemäße Gefäßprothese mit integrierter Aortenklappe weist einen aortenseitigen ersten Abschnitt, einen herz(kammer-)seitigen zweiten Abschnitt und einen zwischen diesen beiden Abschnitten angeordneten dritten Abschnitt auf. Der aortenseitige Abschnitt kann dabei auch als distaler Abschnitt und der herzseitige Abschnitt als proximaler Abschnitt bezeichnet werden. Die in der Medizin üblichen Bezeichnungen "distal" und "proximal" sind dabei in Bezug auf das Herz bzw. die Herzkammer als "vom Herzen weg gerichtet" bzw. "zum Herzen hin gerichtet" zu verstehen.

Der dritte Abschnitt der erfindungsgemäßen Gefäßprothese ist grundsätzlich rohrförmig ausgestaltet. Er weist vorzugsweise als Bulbus-Abschnitt mindestens eine bauchige, beispielsweise auch zwiebelförmige Aufweitung auf. Diese bauchigen Aufweitungen können wie bei der natürlichen Aorta vorgesehen sein und zwar in Form von drei in Umfangsrichtung ausgebildeten Einzelaufweitungen, die den drei Sinusaortae (auch Sinus valsalvae genannt) entsprechen. Diese drei Sinus nehmen die Klappensegel bei geöffneter Herzklappe auf.

Die erfindungsgemäße Gefäßprothese der beschriebenen Art ist dadurch gekennzeichnet, dass ein weiterer ringartiger oder rohrartiger Abschnitt vorgesehen ist. Dieser ist mit der Gefäßprothese verbunden und erstreckt sich im Inneren der Gefäßprothese mindestens im dritten Abschnitt, vorzugsweise Bulbus-Abschnitt mindestens teilweise koaxial zur Gefäßprothese. Dabei ist die in die Gefäßprothese integrierte Aortenklappe aus diesem ringartigen oder rohrartigen Abschnitt ausgebildet.

Mit anderen Worten ist bei der erfindungsgemäßen Gefäßprothese neben den äußeren Prothesenabschnitten ein zusätzlicher innerer Prothesenabschnitt vorhanden. Dieser ist ringartig oder rohrartig ausgebildet und mit den äußeren Prothesenabschnitten verbunden. Aus diesem zusätzlichen inneren Abschnitt ist die Aortenklappe ausgebildet.

Erfindungsgemäß erfolgt die Ausbildung der Aortenklappe dadurch, dass der ringartige oder rohrartige Abschnitt am dritten Abschnitt, vorzugsweise Bulbus-Abschnitt in geeigneter Weise befestigt ist. Insbesondere erfolgt diese Befestigung in dem Bereich der Gefäßprothese, an dem der dritte Abschnitt in den aortenseitigen Abschnitt übergeht.

Die Befestigung ist dabei so vorgesehen, dass die drei Segel der Aortenklappe ausgebildet werden und diese Segel sich im geöffneten Zustand der Klappe an den dritten Abschnitt von innen anlegen oder vorzugsweise in den Bulbus-Abschnitt, insbesondere in drei dort vorhandene Aufweitungen einlegen können.

Die Befestigung (des ringartigen oder rohrartigen Abschnitts) erfolgt an drei Stellen oder drei Bereichen, die in Umfangsrichtung des dritten Abschnitts um jeweils ca. 120° voneinander beabstandet sind. Dementsprechend reicht die Befestigung an diesen drei Stellen oder Bereichen aus, um eine voll funktionsfähige Herzklappe an der erfindungsgemäßen Gefäßprothese auszubilden.

Die genannten drei Stellen bzw. Bereiche befinden sich dementsprechend vorzugsweise dort, wo sich bei der natürlichen Aorta bzw. Aortenklappe die sogenannten Kommissuren, d.h. die Vereinigungsstellen bzw. -bereiche zweier benachbarter Klappensegel befinden. Die Befestigungen selbst entsprechen also bei der erfindungsgemäßen Prothese den Kommissuren bei der natürlichen Aorta/Aortenklappe.

Die genannten drei Stellen bzw. Bereiche sind dabei zweckmäßig so gewählt, dass sie sich bei Vorhandensein von drei bauchigen Aufweitungen im Bulbus-Abschnitt jeweils im Bereich des Übergangs einer bauchigen Aufweitung in die benachbarte bauchige Aufweitung befinden. Dadurch ist gewährleistet, dass die durch die Befestigung(en) ausgebildeten Klappensegel problemlos in diese Aufweitungen passen.

Erfindungsgemäß handelt es sich bei der Befestigung um eine Naht, wobei diese Naht aus einem einzelnen Einfach- oder Mehrfachknoten, aus mehreren solcher Knoten oder aus einer Vielzahl solcher Knoten bestehen kann.

Im Zusammenhang mit den bisherigen Ausführungen zu der beanspruchten Gefäßprothese ist entscheidend, ob mit Verwirklichung der diskutierten Merkmale eine funktionsfähige Aortenklappe in der Gefäßprothese ausgebildet wird. Dies gilt insbesondere für die Verwirklichung der Befestigung des ringartigen oder rohrartigen Abschnitts am dritten Abschnitt. Wichtig ist dabei letzten Endes nur, dass die Aortenklappe sich bei der Systole öffnet und bei der Diastole ausreichend dicht schließt.

Dabei können zur Verbesserung der an sich vorhandenen Funktion der Klappe gegebenenfalls zusätzliche Merkmale (einzeln oder in Kombination miteinander) vorgesehen sein.

So kann gegebenenfalls der ringartige oder rohrartige Abschnitt eine geringere Materialstärke (Materialdicke) aufweisen, als die übrigen Abschnitte der Gefäßprothese. Auf diese Weise lässt sich entweder dieser Abschnitt leichter am dritten Abschnitt, vorzugsweise Bulbus-Abschnitt befestigen oder die daraus gebildeten Klappensegel lassen sich leichter öffnen und schließen oder es ist beides der Fall.

Weiter lassen sich die zuletzt genannten Vorteile gegebenenfalls auch dadurch erreichen, dass das Material, aus dem der ringartige oder rohrartige Abschnitt gebildet ist, eine höhere Elastizität aufweist als das Material, aus dem die übrigen Abschnitte der Gefäßprothese gebildet sind.
Im Zusammenhang mit den beiden zuletzt beschriebenen Merkmalen (Materialstärke und Elastizität des ringartigen oder rohrartigen Abschnitts) kann es ausreichen, wenn diese Merkmale nur in dem Teil dieses Abschnitts verwirklicht sind, an dem die Befestigung erfolgt.

Weiter kann es von Vorteil sein, wenn sich der ringartige oder rohrartige Abschnitt an dem Ende, das zur Befestigung am dritten Abschnitt vorgesehen ist, aufweitet, d.h. in seinem Durchmesser vergrößert. Auch auf diese Weise kann die Befestigung erleichtert und/oder die Klappenfunktion verbessert sein.

Wie bereits erwähnt, kann die Befestigung des ringartigen oder rohrartigen Abschnitts punktuell, d.h. nur an wenigen, beispielsweise nur an drei Stellen oder Bereichen erfolgen. Dies ist jedoch nicht zwingend, so dass es auch möglich ist, eine oder mehrere Nähte variabler Länge auszuführen.

Auch die Lage der Befestigung bzw. der Befestigungen ist grundsätzlich frei wählbar, solange nur durch die Befestigung(en) eine Klappe mit ausreichender Funktion ausgebildet wird.

In Weiterbildung ist es bei der erfindungsgemäßen Prothese bevorzugt, wenn der ringartige oder rohrartige Abschnitt mit der Gefäßprothese am herzseitigen Abschnitt verbunden ist. Vorzugsweise ist diese Verbindung dann im Bereich des Übergangs des herzseitigen Abschnitts in den dritten, vorzugsweise den Bulbus-Abschnitt realisiert.

In allen Fällen erfolgt die Verbindung insbesondere dadurch, dass der ringartige oder rohrartige Abschnitt direkt an den entsprechenden Stellen angeformt ist. Dies kann - wie später beschrieben - unmittelbar bei der Herstellung der Gefäßprothese erfolgen.

Bei den zuletzt beschriebenen Ausführungsformen ist es weiter bevorzugt, wenn die Verbindung von ringartigem oder rohrartigem Abschnitt mit der Gefäßprothese flächig erfolgt. Dies bedeutet, dass die Verbindung nicht nur an einzelnen Stellen, beispielsweise an einzelnen Klebestellen realisiert ist, sondern über größere Flächenbereiche an den jeweiligen Abschnitten. Vorzugsweise erfolgt die Verbindung von ringartigem oder rohrartigem Abschnitt mit der Gefäßprothese in Umfangsrichtung vollflächig. Dies wird im Zusammenhang mit dem Beispiel und den Figuren noch näher erläutert.

In Weiterbildung ist der ringartige oder rohrartige Abschnitt mit der Gefäßprothese bei der Erfindung vorzugsweise monolithisch verbunden. Dies bedeutet, dass durch eine solche Verbindung eine nicht trennbare Einheit zwischen den entsprechenden Abschnitten der Gefäßprothese gebildet ist. Auch hier wird auf das Beispiel und die Figuren verwiesen.

In der Regel ist der ringartige oder rohrartige Abschnitt bei der Erfindung zylinderförmig, mit einem über seine Länge im Wesentlichen konstanten Durchmesser. Mit Vorteil kann es jedoch auch vorgesehen sein, dass auch der ringartige oder rohrartige Abschnitt entlang seiner Länge einen bulbusartigen Abschnitt, vorzugsweise mit drei dort vorgesehenen Sinus aufweist. Dieser (innere) bulbusartige Abschnitt ist insbesondere so vorgesehen, dass er nach der Verbindung des ringartigen oder rohrartigen Abschnitts mit der Gefäßprothese innerhalb des Bulbus-Abschnitts der (äußeren) Gefäßprothese angeordnet ist. Auf diese Weise ist innerhalb des (gegebenenfalls zwiebelartigen) Bulbus-Abschnitts der (äußeren) Gefäßprothese ein weiterer gegebenenfalls zwiebelartiger Bulbus-Abschnitt im Inneren der Gefäßprothese vorgesehen. Eine solche Ausgestaltung des ringartigen oder rohrartigen Abschnitts kann gegebenenfalls eine günstigere Form der Klappensegel und damit eine höhere Dichtigkeit der Aortenklappe ermöglichen.

Bei den zuletzt beschriebenen Ausführungsformen mit einer bulbusartigen Erweiterung des ringartigen oder rohrartigen Abschnitts kann die bauchige Form, z.B. die Zwiebelform dieses Abschnitts gleich, d.h. in gleicher Ausrichtung, wie die bauchige Form, z.B. die Zwiebelform des (äußeren) Bulbus-Abschnitts angeordnet sein oder auch vorzugsweise um 180° verdreht. Dementsprechend kommt bei der zuletzt genannten Ausführung der Teil der bauchigen Form, z.B. der Zwiebelform am ringartigen oder rohrartigen Abschnitt mit größerem Durchmesser im Bereich des kleineren Durchmessers der bauchigen Form, d.h. der Zwiebelform des äußeren Bulbus-Abschnitts zum Liegen. Auch diese (um 180° gedrehte) Anordnung der Formen kann für die Dichtigkeit der Aortenklappe von Vorteil sein.

Die erfindungsgemäße Gefäßprothese kann aus den unterschiedlichsten Materialien gefertigt sein, wobei verschiedene Abschnitte der Prothese ebenfalls aus unterschiedlichen Materialien bestehen können. Vorzugsweise ist die Gefäßprothese insgesamt jedoch nur aus einem Material gefertigt.

Vorzugsweise handelt es sich bei dem Material, aus dem die Gefäßprothese aufgebaut ist, um einen Kunststoff, insbesondere um einen nichtresorbierbaren Kunststoff. Ein solches Material ist vorzugsweise Polyurethan (PUR). Dessen Eigenschaften und Verwendbarkeit in der Medizintechnik sind dem Fachmann ohne weiteres bekannt.

Bezüglich der verwendbaren Polyurethane und anderer einsetzbarer Kunststoffmaterialien wird auf den Inhalt der eingangs erwähnten EP-A2-1579827 verwiesen.

Mit Vorteil ist die erfindungsgemäße Gefäßprothese als Vlies ausgebildet. Vorzugsweise kommt als Vliesmaterial Polyurethan zum Einsatz.

Bei einem Vlies handelt es sich bekanntlich um ein textiles Flächengebilde aus einzelnen Fasern, hier insbesondere Kunststofffasern. Es handelt sich also nicht um ein Gewebe oder Gewirk. Für solche Vliese wird im Englischen auch der Ausdruck "nonwoven" verwendet.

Derartige Vliese können insbesondere als sogenannte Sprühvliese hergestellt sein. Dabei werden (in der Regel verdünnte) Lösungen des entsprechenden Materials, beispielsweise Polyurethan auf eine Unterlage unter Ausbildung des Sprühvlieses aufgesprüht. In diesem Zusammenhang bekannt ist auch die Melt-Blown-Technik, bei dem aufgeschmolzenes Kunststoffmaterial über eine Düse ausgesprüht wird. Auch dies führt zu den genannten Sprühvliesen.

Je nach Herstellungstechnik und Einsatzgebiet kann die erfindungsgemäße Gefäßprothese dicht oder als poröse Prothese ausgebildet sein. Im letzteren Fall ist die Gefäßprothese vorzugsweise mikroporös ausgebildet. Mikroporosität soll dabei bedeuten, dass das Kunststoffmaterial Poren mit Dimensionen < 100 µm besitzt. Dabei sind Porengrößen zwischen 0,1 µm und 100 µm bevorzugt. Solche Porengrößen sind beispielsweise für PUR-Sprühvliese üblich. Dabei ist die Porosität in der Regel offenporig, dass heißt die Poren im Material sind von außen zugänglich.

Erfindungsgemäß kann die Mikroporosität der Gefäßprothese variiert werden. So kann beispielsweise an den nach innen gerichteten Oberflächen der Prothese eine kleinere Porengröße vorgesehen sein als an den nach außen gerichteten Oberflächen. Vorzugsweise kann dabei die Porengröße von den nach innen gerichteten Oberflächen hin zu den nach außen gerichteten Oberflächen im Wesentlichen gleichförmig zunehmen.

Das Volumen der Poren bei einer mikroporösen Gefäßprothese liegt insbesondere zwischen ca. 10 Vol.-% und ca. 80 Vol.-%, bezogen auf das Gesamtvolumen der Gefäßprothese. Vorzugsweise beträgt das Porenvolumen 30 Vol.-% bis 80 Vol.-%, insbesondere 45 Vol.-% bis 55 Vol.-%.

Wahlweise können auf den Innenseiten und/oder Außenseiten der Gefäßprothese zusätzliche Schichten aufgebracht sein. Hierbei kann es sich beispielsweise um Sperrschichten handeln, um Teile der Prothese oder die Gesamtprothese gegen den Zutritt von Flüssigkeiten abzudichten.

In diesem Zusammenhang ist es auch möglich, zusätzliche Funktionsschichten auf die Innenseiten und/oder Außenseiten der Gefäßprothese aufzubringen. Diese können beispielsweise Wirkstoffe wie antimikrobielle Wirkstoffe (z.B. Silber) oder Medikamente (z.B. Antibiotika, Antithrombotika und dergleichen) enthalten. Gegebenenfalls ist es auch möglich, derartige Wirkstoffe direkt als Additive in das Kunststoffmaterial, aus dem die Gefäßprothese gebildet ist, einzubringen.

Die (Mikro-)Porosität lässt sich bei der erfindungsgemäßen Gefäßprothese auch nutzen, um eine Besiedelung der Gefäßprothese mit Zellen zu ermöglichen. Eine solche Besiedelung mit Zellen, insbesondere patienteneigenen Zellen ist insbesondere auf den inneren Oberflächen der Prothese von Vorteil.

Weiter umfasst die Erfindung ein Verfahren zur Herstellung einer Gefäßprothese mit integrierter Aortenklappe, insbesondere zur Herstellung der oben beschriebenen erfindungsgemäßen Gefäßprothese.

Bei dem erfindungsgemäßen Verfahren wird zunächst ein erster Ring oder ein erstes Rohr bereitgestellt oder gefertigt. Dieser Ring bzw. dieses Rohr bildet oder trägt dann später den ringartigen oder rohrartigen Abschnitt, der sich im Inneren der Gefäßprothese zur Ausbildung der Aortenklappe befindet.

Dann wird erfindungsgemäß über diesem ersten Ring oder diesem ersten Rohr ein zweites Rohr, das vorzugsweise mindestens eine bauchige Aufweitung aufweist, angeordnet oder gefertigt. Dies erfolgt derart, dass sich der erste Ring oder das erste Rohr im Inneren des zweiten Rohrs mindestens teilweise koaxial erstreckt.

Weiter wird der erste Ring oder das erste Rohr mit dem zweiten Rohr verbunden, und zwar unter Ausbildung eines aortenseitigen ersten Abschnitts, eines herzseitigen zweiten Abschnitts und eines zwischen diesen beiden Abschnitten angeordneten dritten Abschnitts, der vorzugsweise als Bulbus-Abschnitt mindestens eine bauchige Aufweitung aufweist. Damit ist das Gerüst der Gefäßprothese, mit Ausnahme der (fertigen) Aortenklappe hergestellt.

Anschließend wird dann aus dem ringartigen oder rohrartigen Abschnitt, der nach Verbindung des ersten Rings oder des ersten Rohrs mit dem zweiten Rohr verbleibt, eine Aortenklappe ausgebildet.
Letzteres erfolgt erfindungsgemäß dadurch, dass der ringartige oder rohrartige Abschnitt am dritten Abschnitt (unter Ausbildung der drei Segel der Aortenklappe) befestigt wird. Dies geschieht vorzugsweise so, dass Aufweitungen im als Bulbus-Abschnitt ausgebildeten dritten Abschnitt die Segel der Aortenklappe im geöffneten Zustand der Klappe aufnehmen können.

Außerdem wird bei dem erfindungsgemäßen Verfahren der ringartige oder rohrartige Abschnitt vorzugsweise an drei Stellen oder Bereichen des dritten Abschnitts befestigt, wobei die genannten drei Stellen bzw. Bereiche in Umfangsrichtung des dritten Abschnitts um jeweils ca. 120 ° voneinander beabstandet sind.

Die Befestigung erfolgt durch Vernähen, d.h. durch Anbringen einer Naht. Diese Naht kann wahlweise aus einem einzelnen Einfach- oder Mehrfachknoten, aus mehreren solcher Knoten oder aus einer Vielzahl solcher Knoten bestehen.

Bei allen genannten Ausführungsformen des erfindungsgemäßen Verfahrens ist der erste Ring oder das erste Rohr mit dem zweiten Rohr insbesondere flächig verbunden. Alternativ oder zusätzlich kann es sich bei dieser Verbindung um eine monolithische Verbindung handeln. Auf die entsprechenden bisherigen Ausführungen wird hier ausdrücklich Bezug genommen und verwiesen.

Bei besonders bevorzugten Ausführungen wird das erfindungsgemäße Verfahren so durchgeführt, dass das zweite Rohr über dem ersten Ring oder dem ersten Rohr unter gleichzeitiger Ausbildung der Verbindung zwischen dem ersten Ring oder ersten Rohr mit dem zweiten Rohr gefertigt wird. Auf diese Weise kann ein zusätzlicher Verfahrensschritt, bei dem ein separat bereitgestelltes oder gefertigtes zweites Rohr mit dem ersten Ring/ersten Rohr verbunden wird, eingespart werden.

Vorzugsweise wird bei der Erfindung der erste Ring oder das erste Rohr und das zweite Rohr nach dem gleichen (Herstellungs-)Verfahren gefertigt. Auf diese Weise lässt sich die gleiche Apparatur sowohl für die Herstellung des ersten Rings/des ersten Rohrs als auch des zweiten Rohrs verwenden.

Besonders geeignet für die Herstellung des ersten Rings bzw. des ersten Rohrs und des zweiten Rohrs ist bei dem erfindungsgemäßen Verfahren ein sogenanntes Melt-Blown-Verfahren oder ein (anderes) Sprühverfahren. Diese Verfahren sind besonders geeignet zur Bereitstellung von (Sprüh-)Vliesen, wie sie gerade auch im Hinblick auf diese Herstellungstechniken bereits oben beschrieben sind. Auch auf diese Ausführungen wird ausdrücklich Bezug genommen und verwiesen.
Die Vorteile der genannten Verfahren kommen bei der Erfindung auch deshalb besonders zum Tragen, da sich dadurch bereits bei der Herstellung des zweiten Rohrs die Verbindung dieses Rohrs mit dem ersten Ring/ersten Rohr realisieren lässt. Bei der Herstellung des zweiten Rohrs wird gleichzeitig die Verbindung zwischen diesem zweiten Rohr und dem ersten Ring/ersten Rohr ausgebildet. Dies geschieht, wie später noch näher erläutert wird, dadurch, dass der erste Ring bzw. das erste Rohr nur teilweise mit einer Schutzhülle abgedeckt wird. Dann kann durch Aufsprühen des entsprechenden Materials über dieser Schutzhülle ein nicht mit dem ersten Ring/ersten Rohr verbundenes zweites Rohr ausgebildet werden, während im nicht abgedeckten Bereich des ersten Rings/ersten Rohrs eine vollflächige Verbindung der beiden Bauteile (erster Ring/erstes Rohr bzw. zweites Rohr) erfolgt.

Wie aus den bisherigen Erläuterungen zum erfindungsgemäßen Verfahren bereits teilweise hervorgeht, ist es bevorzugt, wenn die Gefäßprothese im Wesentlichen vollständig aus einem nicht resorbierbaren Kunststoff gefertigt wird. Dabei kann es sich insbesondere um ein Polyurethan (PUR) handeln. Auf die entsprechenden Ausführungen zur Gefäßprothese selbst wird Bezug genommen und verwiesen.

Wie aus den bisherigen Erläuterungen hervorgeht, ist die Bereitstellung der erfindungsgemäßen Gefäßprothese bzw. die Durchführung des erfindungsgemäßen Verfahrens mit Vorteilen gegenüber dem Stand der Technik verbunden.

So ist der Aufbau der erfindungsgemäßen Gefäßprothese vergleichsweise einfach. Im Inneren der eigentlichen Gefäßprothese ist ein ringartiger oder rohrartiger Abschnitt vorgesehen, aus dem die Aortenklappe ausgebildet ist. Zusätzliche Bauelemente, wie beispielsweise Stützelemente sind für die Funktionsfähigkeit der Gefäßprothese (mit integrierter Aortenklappe) nicht erforderlich.

Dieser vergleichsweise einfache Aufbau der Gefäßprothese steht im Zusammenhang mit dem erfindungsgemäßen Verfahren. Durch dieses Verfahren lässt sich die Gefäßprothese in vergleichsweise wenigen Verfahrensschritten herstellen, wobei vorzugsweise Techniken, die grundsätzlich bekannt sind, eingesetzt werden können. Dies resultiert insgesamt in einer vergleichsweise schnellen und kostengünstigen Herstellung der Prothesen.

Schließlich wird dem Chirurgen eine voll einsatzfähige Prothese zur Verfügung gestellt, die er abgesehen von üblicherweise vorgenommenen individuellen Anpassungen, direkt beim chirurgischen Eingriff einsetzen kann.

Weitere Merkmale, Einzelheiten und Vorteile der vorliegenden Erfindung ergeben sich aus der nachfolgenden Beschreibung bevorzugter Ausführungsformen anhand des Beispiels und der Figuren in Kombination mit den Unteransprüchen. Hierbei können die beschriebenen Merkmale jeweils für sich allein oder in Kombination miteinander verwirklicht sein. Das Beispiel und die Figuren dienen lediglich zur Erläuterung der Erfindung und sind in keiner Weise einschränkend zu verstehen.

In den schematischen Figuren zeigen:
- Figur 1:: eine erfindungsgemäße Gefäßprothese mit integrierter Aortenklappe in schematischer Darstellung,
- Figur 2:: die Herstellungsschritte für eine erste erfindungsgemäße Gefäßprothese in schematischer Darstellung,
- Figur 3:: die Herstellungsschritte für eine zweite erfindungsgemäße Gefäßprothese in schematischer Darstellung.

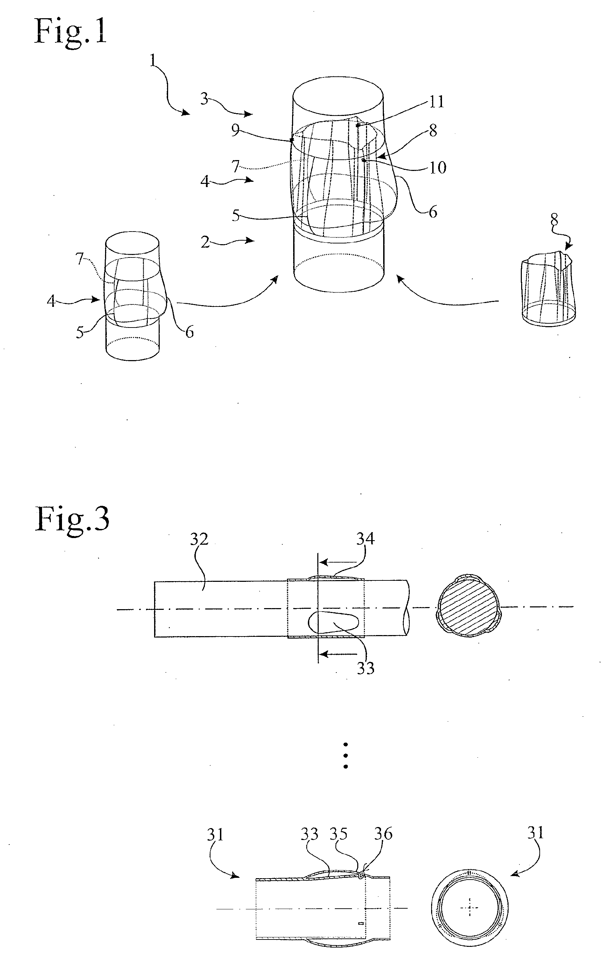

Figur 1 zeigt eine erfindungsgemäße Gefäßprothese 1 mit integrierter Aortenklappe. Diese Gefäßprothese 1 besteht aus einem herz (kammer-)seitigen Abschnitt 2, einem aortenseitigen Abschnitt 3 und einem zwischen diesen beiden Abschnitten 2, 3 angeordneten (dritten) Bulbus-Abschnitt 4. Dieser Bulbus-Abschnitt 4 umfasst drei bauchige Aufweitungen, die in Umfangsrichtung des Bulbus-Abschnitts 4 um jeweils 120° zueinander versetzt angeordnet sind. Diese Aufweitungen sind in ihrer hier zwiebelartigen Form den entsprechenden Aufweitungen der natürlichen Aorta nachgebildet und können somit die Klappensegel der integrierten Aortenklappe der Gefäßprothese 1 im geöffneten Zustand dieser Klappe aufnehmen. Die um circa 120° versetzte Anordnung der drei bauchigen Aufweitungen ist in Figur 1 auch durch den Verlauf der Linien 5, 6 und 7 verdeutlicht.

Weiter ist in Figur 1 ein ringartiger bzw. rohrartiger Abschnitt 8 zu erkennen, der an den herzseitigen Abschnitt 2 angeformt ist und sich im Inneren des Bulbus-Abschnitts 4 in Richtung des aortenseitigen Abschnitts 3 erstreckt. Dieser Abschnitt 8 ist an drei Stellen im Bereich des Übergangs des Bulbus-Abschnitts 4 in den aortenseitigen Abschnitt 3 mit Hilfe von drei Nähten (Nahtpunkten) 9, 10 und 11 befestigt. Diese drei Nähte 9 bis 11 sind in Umfangsrichtung der Gefäßprothese um jeweils circa 120° zueinander versetzt.

Durch diese Befestigung des Abschnitts 8 mit Hilfe der Nähte 9 bis 11 ist die integrierte Aortenklappe in der Gefäßprothese 1 ausgebildet. Die daraus resultierenden Klappensegel legen sich bei dem Schließen dieser Klappe aneinander und sorgen auf diese Weise für einen ausreichend dichten Verschluss der Klappe.

Aus Gründen der Übersichtlichkeit sind in Figur 1 der äußere Teil der Gefäßprothese 1 (links unten) und der innere Teil der Gefäßprothese 1 (rechts unten) getrennt dargestellt. Durch diese Darstellung lässt sich die Funktionsweise dieser beiden Teile besser erkennen. Die ausschließlich zum besseren Verständnis gezählte Darstellung soll natürlich nicht bedeuten, dass diese beiden Teile getrennt vorliegen.

Die in Figur 1 dargestellte Gefäßprothese 1 ist, wie im Beispiel noch erläutert als Sprühvlies aus Polyurethan (PUR) gefertigt. Sie ist frei von zusätzlichen Stütz- oder Abstandselementen.

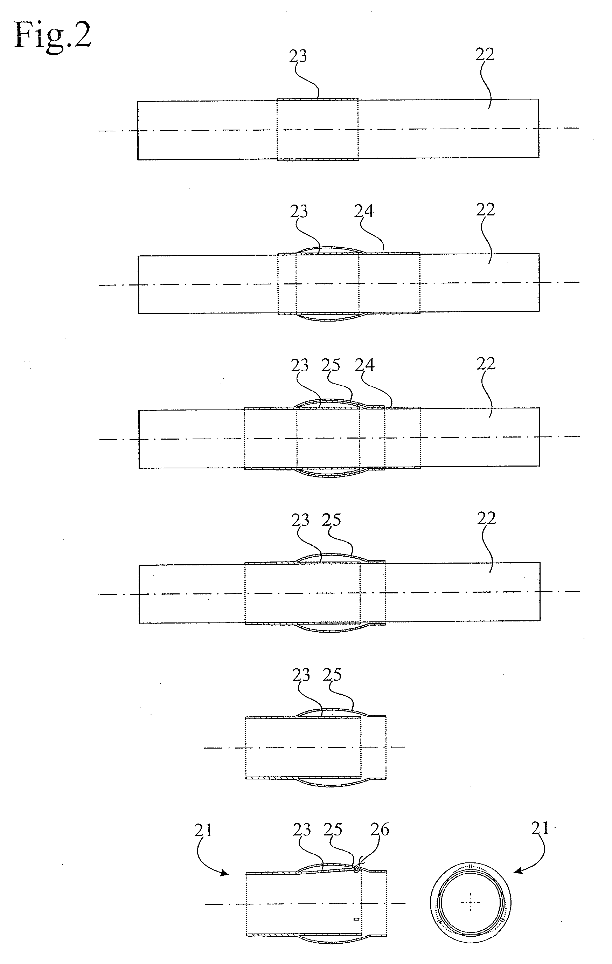

Gemäß Figur 2 sind von oben nach unten Verfahrensschritte für die Herstellung einer ersten erfindungsgemäßen Gefäßprothese 21 dargestellt. Die auf diese Weise hergestellte Gefäßprothese 21 entspricht in ihrem Aufbau weitgehend der Gefäßprothese 1 wie sie in Figur 1 offenbart ist.

Nach der Vorgehensweise in Figur 2 wird zunächst auf einer zylinderförmigen Halterung 22 ein rohrartiger Abschnitt 23 der späteren Gefäßprothese 21 bereitgestellt. Aus diesem Abschnitt 23, der Abschnitt 8 von Figur 1 entspricht, wird später die Aortenklappe ausgebildet.

Das Bereitstellen von Abschnitt 23 kann beispielsweise durch Aufsprühen einer Polyurethanlösung auf die Halterung 22 erfolgen, so dass es sich bei dem Abschnitt 23 um ein Sprühvlies aus Polyurethan handelt.

Dann wird über dem Abschnitt 23 ein Sprühschutz 24 angeordnet, der mindestens einen Teil des Abschnitts 23 an der Seite, die später den herz(kammer-)seitigen Abschnitt der Gefäßprothese 21 bildet, frei lässt. Hierbei handelt es sich gemäß Figur 2 um die linke Seite des Abschnitts 23. An der anderen (in Figur 2 rechten) Seite des Abschnitts 23 ragt der Sprühschutz 24 über den Abschnitt 23 hinaus.

Darüber hinaus besitzt der Sprühschutz 24 drei bauchige Aufweitungen, die in seiner Umfangsrichtung um jeweils circa 120° zueinander versetzt sind. In den entsprechenden Bereichen werden somit später die entsprechenden Aufweitungen des Bulbus-Abschnitts der Gefäßprothese 21 ausgebildet.

Anschließend werden der frei liegende Teil des Abschnitts 23 und der Sprühschutz 24 mit Polyurethan übersprüht. Das so erhaltene Konstrukt ist in der dritten Abbildung von oben in Figur 2 dargestellt. Dort sind deutlich übereinander der (innere) Rohrabschnitt 23, der Sprühschutz 24 und der äußere Teil 25 der Gefäßprothese zu erkennen.

Die darunter liegende Abbildung von Figur 2 zeigt den Herstellungszustand der Gefäßprothese 21 nach Entfernen des Sprühschutzes 24. Der innere Teil der bis dahin gefertigten Gefäßprothese 21 wird durch Rohrabschnitt 23 gebildet, der an seiner linken herz(kammer-)seitigen Seite mit dem äußeren Teil 25 der Gefäßprothese durch den Sprühvorgang fest (einstückig) verbunden ist. Der äußere Teil 25 weist die durch den Sprühschutz 24 vorgegebenen bauchigen (hier zwiebelförmigen) Aufweitungen auf, die den Bulbus-Abschnitt der Gefäßprothese bilden. Dieser Bulbus-Abschnitt geht an der rechten Seite der Darstellung in den aortenseitigen Abschnitt der Gefäßprothese 21 über.

Im nächsten Schritt wird die bis dahin hergestellte Gefäßprothese von der Halterung abgenommen. Der daraus resultierende Zustand ist in Figur 2 in der zweiten Abbildung von unten dargestellt.

Dann wird der Rohrabschnitt 23, der sich im Bulbus-Abschnitt des äußeren Teils 25 koaxial erstreckt, im Bereich des Übergangs des Bulbus-Abschnitts in den aortenseitigen Abschnitt befestigt. Dies erfolgt mit Hilfe von drei Nähten 26, die in Umfangsrichtung der Gefäßprothese um jeweils circa 120 ° voneinander beabstandet sind. Von diesen drei Nähten ist in Figur 2, unterste Abbildung eine Naht explizit dargestellt.

Rechts neben der unteren Abbildung findet sich noch eine Draufsicht auf die Gefäßprothese 21 (in Blickrichtung von rechts dieser Abbildung).

Es ist klar zu erkennen, dass die beiden untersten Darstellungen in Figur 2 im Wesentlichen den Aufbau der Gefäßprothese 1 gemäß Figur 1 wiedergeben. Insoweit sind die Offenbarungen der Figuren 1 und 2 im Zusammenhang zu sehen.

Die Herstellung einer zweiten erfindungsgemäßen Gefäßprothese 31 ist in Figur 3 dargestellt. Da eine ganze Reihe von Verfahrensschritten den Verfahrensschritten gemäß Figur 2 entspricht, wird insoweit über die senkrecht gepunktete Linie in Figur 3 auf diese Verfahrensschritte der Figur 2 (und die dazugehörige Erläuterung) Bezug genommen und verwiesen.

Auch in Figur 3 wird von einer zylindrischen Halterung 32 ausgegangen. Diese Halterung 32 ist allerdings so ausgebildet, dass sie auf ihrer Oberfläche drei bauchige (hier zwiebelförmige) Erhebungen aufweist, die in Umfangsrichtung jeweils um circa 120 ° zueinander versetzt sind. Von diesen drei Erhebungen ist in der oberen Abbildung von Figur 3 die Erhebung 33 explizit dargestellt. Durch diese Erhebungen wird erreicht, dass auch der zunächst bereitgestellte (innere) ringartige oder rohrartige Abschnitt 34 bauchige Aufweitungen nach Art der Sinus der natürlichen Aorta aufweist. Dies erläutert auch die rechte Darstellung der oberen Abbildung von Figur 3. Diese stellt eine Schnittansicht entlang der mit Pfeilen gekennzeichneten Linie in der linken Abbildung dar, wobei hier der (innere) Rohrabschnitt 34 bereits ausgebildet ist.

Wie im Zusammenhang mit Figur 2 bereits erläutert, wird der Rohrabschnitt 34 vorzugsweise durch Aufsprühen einer Polyurethanlösung als Sprühvlies auf der Halterung 32 ausgebildet. Wie erwähnt besitzt der Rohrabschnitt 34 im Gegensatz zu der Ausführung gemäß Figur 2 bereits bauchige, hier zwiebelförmige Erweiterungen. Diese Erweiterungen, die den Erhebungen auf der Halterung 32 entsprechen, sind gegenüber den später im äußeren Teil der Gefäßprothese 31 ausgebildeten bauchigen Aufweitungen nicht notwendigerweise, aber vorzugsweise um 180 ° verdreht. Betrachtet man diese Aufweitungen ausgehend von der hier gewählten Zwiebelform, so kommen dementsprechend die beiden Zwiebelformen des inneren Rohrabschnitts und des äußeren Prothesenabschnitts um 180 ° gegeneinander versetzt (verdreht) zu liegen. Das heißt der bauchige Teil mit größerem Volumen der einen Aufweitungen kommt über bzw. unter den weniger bauchigen Teilen der anderen Aufweitungen zu liegen und umgekehrt. Diese Vorgehensweise wurde bereits im allgemeinen Teil der Beschreibung dargestellt.

Die sich nach Ausbildung des Abschnitts 34 anschließenden Verfahrensschritte (Anordnen des Sprühschutzes, Bereitstellen (Sprühen) des äußeren Teils, Entfernen des Sprühschutzes und Abnehmen der bis dahin gebildeten Gefäßprothese von der Halterung) entsprechen den Verfahrensschritten gemäß Figur 2. Deshalb kann hier auf diese Abbildungen und die zugehörigen Erläuterungen Bezug genommen und verwiesen werden.

Abschließend wird dann auch gemäß Figur 3 (siehe untere Abbildung) der Abschnitt 34 am äußeren Teil 35 mit Hilfe von drei Nähten 36 befestigt. Daraus resultiert die Gefäßprothese 31, die in der unteren Abbildung rechts noch in Draufsicht dargestellt ist.

Im Unterschied zu der Gefäßprothese 21 gemäß Figur 2 weist die Gefäßprothese 31 gemäß Figur 3 also einen (inneren) Abschnitt 34 auf, der ebenfalls drei bauchige, hier zwiebelförmige Aufweitungen aufweist. Diese Aufweitungen sind in der unteren Abbildung von Figur 3 nicht explizit dargestellt.

### Beispiel

Zunächst wird eine zylindrische Halterung (Welle) zur Herstellung der Prothese bereitgestellt.

Dann wird mit Hilfe einer Sprühpistole eine Polyurethanlösung (PUR-Lösung) auf die Halterung aufgesprüht. Das Aufsprühen erfolgte in mehreren Zyklen, wobei die Sprühpistole von links nach rechts und wieder zurück mit einem Auftragswinkel von 45 ° über die Halterung hinweg bewegt wird. Die Halterung wird dabei rotiert, um einen gleichförmigen Auftrag unter Ausbildung der rohrförmigen Prothese zu ermöglichen. Der Sprühdruck liegt zwischen 0,1 und wenigen bar. Der Sprühabstand kann zwischen 50 mm und 500 mm variiert werden. Die PUR-Lösung ist beispielsweise 10%ig (in Propanol).

In der beschriebenen Weise wird zunächst eine innere Röhre (Rohr), der innere Rohrabschnitt gesprüht. Wie bereits erläutert wird später aus dieser inneren Röhre die Aortenklappe gebildet.

Nach einem Antrocknen wird diese innere Röhre mit einem Sprühschutz (Latexhülle) überzogen. Dabei wird an einer Seite der Röhre ein Teil dieser Röhre frei gelassen. Hier soll beim Übersprühen die Verbindung der äußeren Röhre mit der inneren Röhre erfolgen. Dieser Verbindungsabschnitt bildet später den herz(kammer-)seitigen Abschnitt der Gefäßprothese.

Wie bereits erläutert weist der Sprühschutz bauchige Aufweitungen auf, die später den Bulbus-Abschnitt der fertigen Gefäßprothese bilden. Es handelt sich, wie erläutert, um drei bauchige, im vorliegenden Fall zwiebelförmige Einzelaufweitungen, die am Außenumfang des Sprühschutzes in Umfangsrichtung (senkrecht zur Achse) um jeweils (circa) 120 ° zueinander beabstandet sind.

Die so erhaltene Anordnung aus innerer Röhre und Sprühschutz wird dann mit der gleichen PUR-Lösung übersprüht, wobei die genannten Verfahrensparameter Anwendung finden. Auf diese Weise entsteht das Konstrukt aus innerer Röhre und mit dieser verbundener Außenröhre (mit ihren drei bauchigen Aufweitungen).

Dann wird nach dem Antrocknen der Sprühschutz entfernt und die bis dahin erhaltene Prothese von der Halterung abgenommen. Diese Prothese besteht nun aus der inneren Röhre, die am herz(kammer-)seitigen Ende mit der äußeren Röhre (einstückig) verbunden ist. Die innere Röhre erstreckt sich im Inneren des Bulbus-Abschnitts der äußeren Röhre. Der Bulbus-Abschnitt der äußeren Röhre geht an dem Ende, das dem herzseitigen Abschnitt abgewandt ist, in einen aortenseitigen Abschnitt über.

Nunmehr wird die innere Röhre an die äußere Röhre angenäht, und zwar im Bereich des Übergangs des Bulbus-Abschnitts in den aortenseitigen Abschnitt. Dies erfolgt mit Hilfe von drei Nähten, die in Umfangsrichtung des Bulbus-Abschnitts um jeweils 120 ° zueinander beabstandet sind.

Damit wird ohne weitere Abstands- oder Stützelemente eine funktionstaugliche Gefäßprothese bereitgestellt. Diese entspricht in ihrem Aufbau im Wesentlichen der Gefäßprothese 1 von Figur 1.

Strömt Blut aus dem Herzen aus, so öffnen sich die zwischen den drei Nähten ausgebildeten Segel der Aortenklappe automatisch und legen sich in die im Bulbus-Abschnitt der Prothese vorgesehenen bauchigen Aufweitungen ein. Dementsprechend ist ein ungehinderter Blutfluss möglich.

Umgekehrt schließt sich die Aortenklappe bei geringem Blutdruck, wenn die Auswurfphase des Herzens beendet ist. Hier können sich die drei Segel der Aortenklappe unter Abdichtung der Klappe aneinander legen, so dass die Klappe ausreichend dicht verschlossen ist.

## Patentansprüche

1. Rohrförmige Gefäßprothese (1) mit integrierter Aortenklappe, die einen aortenseitigen ersten Abschnitt (3), einen herzseitigen zweiten Abschnitt (2) und einen zwischen diesen beiden Abschnitten angeordneten dritten Abschnitt (4) aufweist, **dadurch gekennzeichnet, dass** die Gefäßprothese frei von Verstärkungs- oder Stützelementen ist und ein weiterer ringartiger oder rohrartiger Abschnitt (8) vorgesehen ist, der mit der Gefäßprothese verbunden ist und sich im Inneren der Gefäßprothese mindestens im dritten Abschnitt mindestens teilweise koaxial zur Gefäßprothese erstreckt, und dass die Aortenklappe aus dem ringartigen oder rohrartigen Abschnitt (8) ausgebildet ist, wobei die Aortenklappe durch Befestigung des ringartigen oder rohrartigen Abschnitts (8) am dritten Abschnitt (4), insbesondere im Bereich des Übergangs des dritten Abschnitts (4) in den aortenseitigen Abschnitt (3) ausgebildet ist, und die Befestigung des ringartigen oder rohrartigen Abschnitts (8) an drei Stellen oder Bereichen erfolgt, die in Umfangsrichtung des dritten Abschnitts (4) um jeweils ca. 120° voneinander beabstandet sind, und wobei es sich bei der Befestigung um mindestens eine Naht (9, 10, 11) handelt.

2. Gefäßprothese nach Anspruch 1, **dadurch gekennzeichnet, dass** der dritte Abschnitt (4) als Bulbus-Abschnitt mit mindestens einer bauchigen Aufweitung ausgebildet ist, wobei vorzugsweise drei den Sinus aortae (Sinus Valsalvae) entsprechende Einzelaufweitungen vorgesehen sind.

3. Gefäßprothese mit integrierter Aortenklappe nach Anspruch 1 oder Anspruch 2, **dadurch gekennzeichnet, dass** der ringartige oder rohrartige Abschnitt (8) mit der Gefäßprothese (1) am herzseitigen Abschnitt (2), insbesondere im Bereich des Übergangs des herzseitigen Abschnitts in den dritten Abschnitt verbunden ist, vorzugsweise dort angeformt ist.

4. Gefäßprothese mit integrierter Aortenklappe nach einem der vorhergehenden Ansprüche, **dadurch gekennzeichnet, dass** der ringartige oder rohrartige Abschnitt (8) mit der Gefäßprothese (1) flächig verbunden ist.

5. Gefäßprothese mit integrierter Aortenklappe nach einem der vorhergehenden Ansprüche, **dadurch gekennzeichnet, dass** der ringartige oder rohrartige Abschnitt (8) mit der Gefäßprothese (1) monolithisch verbunden ist.

6. Gefäßprothese mit integrierter Aortenklappe nach einem der vorhergehenden Ansprüche, **dadurch gekennzeichnet, dass** der ringartige oder rohrartige Abschnitt (8) an seinem Umfang ebenfalls mindestens eine bulbusartige Erweiterung aufweist.

7. Verfahren zur Herstellung einer Gefäßprothese mit integrierter Aortenklappe, insbesondere zur Herstellung einer Gefäßprothese (21; 31) nach einem der vorhergehenden Ansprüche, **dadurch gekennzeichnet, dass**
- ein erster Ring oder ein erstes Rohr (23; 34) bereitgestellt oder gefertigt wird,
- über diesem Ring oder diesem Rohr ein zweites Rohr (25; 35), das vorzugsweise mindestens eine bauchige Aufweitung aufweist, angeordnet oder gefertigt wird, derart, dass sich der erste Ring oder das erste Rohr im Inneren des zweiten Rohrs mindestens teilweise koaxial erstreckt,
- der erste Ring oder das erste Rohr (23; 34) mit dem zweiten Rohr (25; 35) verbunden wird, unter Ausbildung eines aortenseitigen ersten Abschnitts (3), eines herzseitigen zweiten Abschnitts (2) und eines zwischen diesen beiden Abschnitten angeordneten dritten Abschnitts (4), der vorzugsweise als Bulbus-Abschnitt mindestens eine bauchige Aufweitung aufweist, und
- aus dem ringartigen oder rohrartigen Abschnitt (8), der nach Verbindung des ersten Rings oder des ersten Rohrs mit dem zweiten Rohr verbleibt, eine Aortenklappe ausgebildet wird, wobei die Aortenklappe durch Befestigung des ringartigen oder rohrartigen Abschnitts (8) am dritten Abschnitt (4) ausgebildet wird, und die Befestigung des ringartigen oder rohrartigen Abschnitts (8) an drei Stellen oder Bereichen des dritten Abschnitts (4) erfolgt, die in Umfangsrichtung des dritten Abschnitts (4) um jeweils ca. 120° voneinander beabstandet sind, und wobei die Befestigung durch Vernähen erfolgt.

8. Verfahren nach Anspruch 7, **dadurch gekennzeichnet, dass** der erste Ring oder das erste Rohr mit dem zweiten Rohr flächig verbunden wird.

9. Verfahren nach Anspruch 7 oder Anspruch 8, **dadurch gekennzeichnet, dass** der erste Ring oder das erste Rohr mit dem zweiten Rohr monolithisch verbunden wird.

10. Verfahren nach einem der Ansprüche 7 bis 9, **dadurch gekennzeichnet, dass** das zweite Rohr über dem ersten Ring oder dem ersten Rohr unter gleichzeitiger Ausbildung der Verbindung zwischen dem ersten Ring oder ersten Rohr mit dem zweiten Rohr gefertigt wird.

11. Verfahren nach einem der Ansprüche 7 bis 10, **dadurch gekennzeichnet, dass** der erste Ring oder das erste Rohr und das zweite Rohr nach dem gleichen Verfahren gefertigt werden.

## Claims

1. Tubular vascular prosthesis (1) with integrated aortic valve, including a first section (3) on the aortic side, a second section (2) on the heart side and a third section (4) disposed between the two aforementioned sections, **characterized in that** the vascular prosthesis is free of reinforcement or support elements and a further annular or tubular type section (8) is provided, which section is connected to the vascular prosthesis and extends in the interior of the vascular prosthesis at least in the third section at least partially coaxially to the vascular prosthesis, and **in that** the aortic valve is composed of the annular or tubular type section (8), wherein the aortic valve is formed by fixing of the annular or tubular type section (8) to the third section (4), in particular in the region of the transition of the third section (4) into the aortic-sided section (3), and fixing of the annular or tubular type section (8) is on three locations or regions which are spaced from each other in the circumferential direction of the third section (4) by in each case approximately 120°, and wherein said fixing is at least one seam (9, 10, 11).

2. Vascular prosthesis according to claim 1, **characterized in that** the third section (4) is a bulbus section having at least one bulgy enlargement, wherein preferably three individual enlargements corresponding to the aortic sinus (sinus aortae, sinus valsalvae) are provided.

3. Vascular prosthesis with integrated aortic valve according to claim 1 or claim 2, **characterized in that** the annular or tubular type section (8) is connected to the vascular prosthesis (1) on the heart-sided section (2), in particular in the region of the transition of the heart-sided section into the third section, and preferably is integrally shaped thereon.

4. Vascular prosthesis with integrated aortic valve according to any of the preceding claims, **characterized in that** the annular or tubular type section (8) is planarly connected to the vascular prosthesis (1).

5. Vascular prosthesis with integrated aortic valve according to any of the preceding claims, **characterized in that** the annular or tubular type section (8) is monolithically connected to the vascular prosthesis (1).

6. Vascular prosthesis with integrated aortic valve according to any of the preceding claims, **characterized in that** the annular or tubular type section (8) also includes at least one bulbus type enlargement on the circumference thereof.

7. Method for producing a vascular prosthesis with integrated aortic valve, in particular for producing a vascular prosthesis (21; 31) according to any of the preceding claims, **characterized in that**
- a first ring or a first tube (23; 34) is provided or manufactured,
- a second tube (25; 35), which preferably has at least one bulgy enlargement, is disposed or manufactured over said ring or said tube in such a manner that the first ring or the first tube extends in the interior of the second tube at least partially coaxially,
- the first ring or the first tube (23; 34) is connected to the second tube (25; 35), while forming a first section (3) on the aortic side, a second section (2) on the heart side and a third section (4) disposed between the two aforementioned sections, the latter preferably including a bulbus section having at least one bulgy enlargement, and
- an aortic valve is formed by the annular or tubular type section (8), which remains after connecting the first ring or the first tube to the second tube, wherein the aortic valve is formed by fixing of the annular or tubular type section (8) to the third section (4), and fixing of the annular or tubular type section (8) is on three locations or regions of the third section (4), which are spaced from each other in the circumferential direction of the third section (4) by in each case approximately 120°, and wherein said fixing is by sewing.

8. Method according to claim 7, **characterized in that** the first ring or the first tube is connected to the second tube in a planar manner.

9. Method according to claim 7 or claim 8, **characterized in that** the first ring or the first tube is connected to the second tube in a monolithic manner.

10. Method according to any of claims 7 to 9, **characterized in that** the second tube is manufactured over the first ring or the first tube with simultaneous formation of the connection between the first ring or the first tube to the second tube.

11. Method according to any of claims 7 to 10, **characterized in that** the first ring or the first tube and the second tube are manufactured using the same method.

## Revendications

1. Prothèse vasculaire (1) de forme tubulaire avec valve aortique intégrée, laquelle possède une première portion (3) côté aorte, une deuxième portion (2) côté coeur et une troisième portion (4) disposée entre ces deux portions, **caractérisée en ce que** la prothèse vasculaire est exempte d'éléments de renforcement ou de support et qu'il existe une portion (8) de type bague ou de type tube qui est reliée à la prothèse vasculaire et qui s'étend à l'intérieur de la prothèse vasculaire, au moins dans la troisième portion et au moins partiellement coaxialement à la prothèse vasculaire, et **en ce que** la valve aortique est formée à partir de la portion (8) de type bague ou de type tube, la valve aortique étant formée en fixant la portion (8) de type bague ou de type tube à la troisième portion (4), notamment dans la zone de la transition de cette troisième portion (4) en la portion côté aorte (3), et la fixation de la portion (8) de type bague ou de type tube s'effectue en trois points ou zones qui sont respectivement espacé(e)s les un(e)s des autres d'environ 120° dans le sens périphérique de la troisième portion (4), et la fixation étant au moins une couture (9, 10, 11).

2. Prothèse vasculaire selon la revendication 1, **caractérisée en ce que** la troisième portion (4) est réalisée sous la forme d'une portion de bulbe ayant au moins un élargissement bombé, trois élargissements correspondant au sinus aortique (sinus de Valsalva) étant de préférence prévus.

3. Prothèse vasculaire avec valve aortique intégrée selon la revendication 1 ou la revendication 2, **caractérisée en ce que** la portion (8) de type bague ou de type tube est reliée à la prothèse vasculaire (1), ou de préférence façonnée, au niveau de la portion côté coeur (2), notamment dans la zone de la transition de la portion côté coeur en la troisième portion.

4. Prothèse vasculaire avec valve aortique intégrée selon l'une des revendications précédentes, **caractérisée en ce que** la portion (8) de type bague ou de type tube est reliée à la prothèse vasculaire (1) à plat.

5. Prothèse vasculaire avec valve aortique intégrée selon l'une des revendications précédentes, **caractérisée en ce que** la portion (8) de type bague ou de type tube est reliée à la prothèse vasculaire (1) de manière monolithique.

6. Prothèse vasculaire avec valve aortique intégrée selon l'une des revendications précédentes, **caractérisée en ce que** la portion (8) de type bague ou de type tube possède également au moins un élargissement de type bulbe sur son pourtour.

7. Procédé de fabrication d'une prothèse vasculaire avec valve aortique intégrée, notamment pour la fabrication d'une prothèse vasculaire (21 ; 31) selon l'une des revendications précédentes, **caractérisé en ce que**
- une première bague ou un premier tube (23 ; 34) est fourni ou fabriqué,
- un deuxième tube (25 ; 35), lequel possède de préférence au moins un élargissement bombé, est disposé ou fabriqué au-dessus de cette bague ou de ce tube de telle sorte que la première bague ou le premier tube s'étend au moins partiellement de manière coaxiale à l'intérieur du deuxième tube,
- la première bague ou le premier tube (23 ; 34) est relié(e) au deuxième tube (25 ; 35) en formant une première portion (3) côté aorte, une deuxième portion (2) côté coeur et une troisième portion (4) disposée entre ces deux portions, laquelle est réalisée sous la forme d'une portion de bulbe possédant de préférence au moins un élargissement bombé, et
- une valve aortique est formée à partir de la portion (8) de type bague ou de type tube qui reste après avoir relié la première bague ou le premier tube au deuxième tube, la valve aortique étant formée en fixant la portion (8) de type bague ou de type tube à la troisième portion (4), et la fixation de la portion (8) de type bague ou de type tube s'effectuant en trois points ou zones de la troisième portion (4) qui sont respectivement espacé(e)s les un(e)s des autres d'environ 120° dans le sens périphérique de la troisième portion (4), la fixation étant réalisée par couture.

8. Procédé selon la revendication 7, **caractérisé en ce que** la première bague ou le premier tube est relié(e) à plat au deuxième tube.

9. Procédé selon la revendication 7 ou la revendication 8, **caractérisé en ce que** la première bague ou le premier tube est relié au deuxième tube de manière monolithique.

10. Procédé selon l'une des revendications 7 à 9, **caractérisé en ce que** le deuxième tube est fabriqué au-dessus de la première bague ou du premier tube en formant simultanément la liaison entre la première bague ou le premier tube et le deuxième tube.

11. Procédé selon l'une des revendications 7 à 10, **caractérisé en ce que** la première bague ou le premier tube et le deuxième tube sont fabriqués selon le même procédé.
